# EUROPEAN PATENT APPLICATION

(11) **EP 1 016 379 A1**
(43) Date of publication of application: **05.07.2000**
(21) Application number: 99310547.7
(22) Date of filing: 23.12.1999
(51) Int. Cl.: A61B 17/135

(54) **Gel tourniquet cuff**

(30) Priority: 31.12.1998 US 114726 P
(71) Applicant: DEPUY ORTHOPAEDICS, INC., Warsaw, Indiana 46581-0988 (US)
(72) Inventor: Guzman, Jose F, Warsaw, IN 46581 (US); Clupper, Christian, Columbia Citc, IN 46725 (US)
(74) Representative: Belcher, Simon James

(57) **Abstract**

A tourniquet cuff (10) includes a first layer (20), a second layer (40), and a third layer (60). The first layer (20) has an inner side (22) impermeable to air and the second layer (40) has a first side (44) impermeable to air. The inner side (22) of the first layer (20) and the first side (44) of the second layer (40) cooperate to define a first cavity (96) formed to receive a gel-like material (15) therein. Further, the third layer (60) has an inner side (64) which cooperates with a second side (42) of the second layer (40) to define a second cavity (94) formed to receive pressurized air. The tourniquet cuff (10) further includes a means (80 and 90) for adjustably securing the tourniquet cuff (10) about a patient's limb such that the gel-like material (15) is positioned between the patient's limb and the second cavity (94) to uniformly and comfortably distribute pressure around the patient's limb.

## Description

The present invention relates to a tourniquet cuff and particularly to a tourniquet cuff for use during surgery which provides uniform pressure distribution and patient comfort.

Tourniquet cuffs are used to reduce the flow of blood to a location on the human body where surgery is taking place. Typically, tourniquet cuffs employ a cotton layer which wraps around the patient's arm or leg at a point proximal to the heart from where surgery is being performed. During use, the cotton layer is positioned adjacent to the patient's limb. An expandable air bladder, then, surrounds the cotton layer to allow various pressures to be applied to the cotton layer, and thus, to the patient's limb. By providing pressure around a patient's limb, the tourniquet cuff reduces the flow of blood to that portion of the limb distal to the tourniquet cuff and toward the limb's extremity. The cotton layer is employed so that the tourniquet cuff may comfortably be placed on the patient's limb.

According to one embodiment of the present invention, a tourniquet cuff is provided to better distribute pressure and for better patient comfort than is provided by traditional tourniquet cuffs. The tourniquet cuff of the present invention includes a gel layer within the tourniquet cuff. Adjacent to the gel layer is an air bladder which is inflated to provide pressure around a patient's limb. As an alternative to an air bladder, other devices, such as a simple strap, may be used to provide pressure around the patient's limb. During use, the air bladder is inflated around the patient's limb with the gel layer positioned between the patient's limb and the air bladder. The gel layer conforms to the particular patient's limb for added comfort and better pressure distribution than may be provided by traditional tourniquet cuffs. Another object of the present invention is to provide a funnel-shaped (tapered) section at one end of the tourniquet to allow the tourniquet to resist folding and buckling and to allow the tourniquet to conform more comfortably to the patient's limb.

In a preferred embodiment, a length of polyester nap material and two lengths of nylon material are positioned in layers and sealed together at their perimeters to create two bladders and define two air-tight chambers -- a first chamber between the polyester nap layer and the middle nylon layer and a second between the middle nylon layer and the outer nylon layer. A gel-like material is positioned within the first chamber and the second chamber can be inflated to provide increased pressure to a patient's limb when the tourniquet is around the patient's limb.

The polyester nap material and the top nylon layer each have a tapered portion, one coupled to the other, each having a wide end and a narrow end, the wide end of the polyester nap material and the wide end of the top nylon layer being coupled to one end of the two bladders. A strap provided by the loop-portion of a hook-and-loop closure material is coupled to the narrow end of the polyester nap material and to the narrow end of the top nylon layer. A corresponding hook-portion strap is attached to the top surface of the second chamber for securing the tourniquet cuff about the patient's limb. A suitable hook-and-loop closure material is that sold under the trade mark VELCRO.

In yet another embodiment, a length of polyester nap material and a first length of nylon material are positioned in layers and sealed together at their perimeters to create a first chamber. A gel-like material is positioned within the first chamber. A second length and a third length of nylon material of substantially the same dimensions as the first chamber are positioned in layers and sealed together at their perimeters and also lengthwise down their approximate middles to create second and third parallel air-tight chambers. The second and the third chambers can be inflated independently of each other to provide increased pressure to a patient's limb when the tourniquet is around the patient's limb.

In yet a further embodiment, an elongated chamber is wrapped and fastened about a patient's limb and inflated to apply pressure to the limb and reduce or eliminate blood flow. An additional chamber is secured to the inflatable chamber and extends along it so that it is disposed between the inflatable chamber and the surface of the patient's limb. A gel-like substance is disposed within the additional chamber to uniformly and comfortably distribute pressure on the patient's limb.

In a further aspect, the invention provides a tourniquet cuff which comprises an elongated inflatable chamber to be wrapped and fastened about a patient's limb and inflated to apply pressure to the limb and reduce or eliminate blood flow; an additional chamber attached to the inflatable chamber to extend along it and to be disposed between the inflatable chamber and the surface of the patient's limb; and a gel-like substance disposed within the additional chamber to uniformly and comfortably distribute pressure on the patient's limb.

Embodiments of the present invention will now be described by way of example with reference to the accompanying drawings, in which
Fig. 1 is a perspective view of the gel tourniquet cuff in accordance with the present invention;
Fig. 2 is a sectional view of the tourniquet of Fig. 1 taken along line 2-2;
Fig. 3 is a sectional view similar to that shown in Fig. 2 illustrating an additional embodiment of the present invention;
Fig. 4 is a sectional view of a bladder port of the tourniquet of Fig. 1 taken along the line 4-4;
Fig. 5 is a plan view of one end of the tourniquet of Fig. 1, showing the loop portion of a hook-and-loop closure material coupled to a polyester nap funnel-shaped end of the tourniquet;
Fig. 6 is a perspective view showing another embodiment of the gel tourniquet cuff in accordance with the present invention;
Fig. 7 is a sectional view of the tourniquet of Fig. 6;
Fig. 8 is a sectional view similar to that shown in Fig. 7 illustrating an additional embodiment of the present invention..

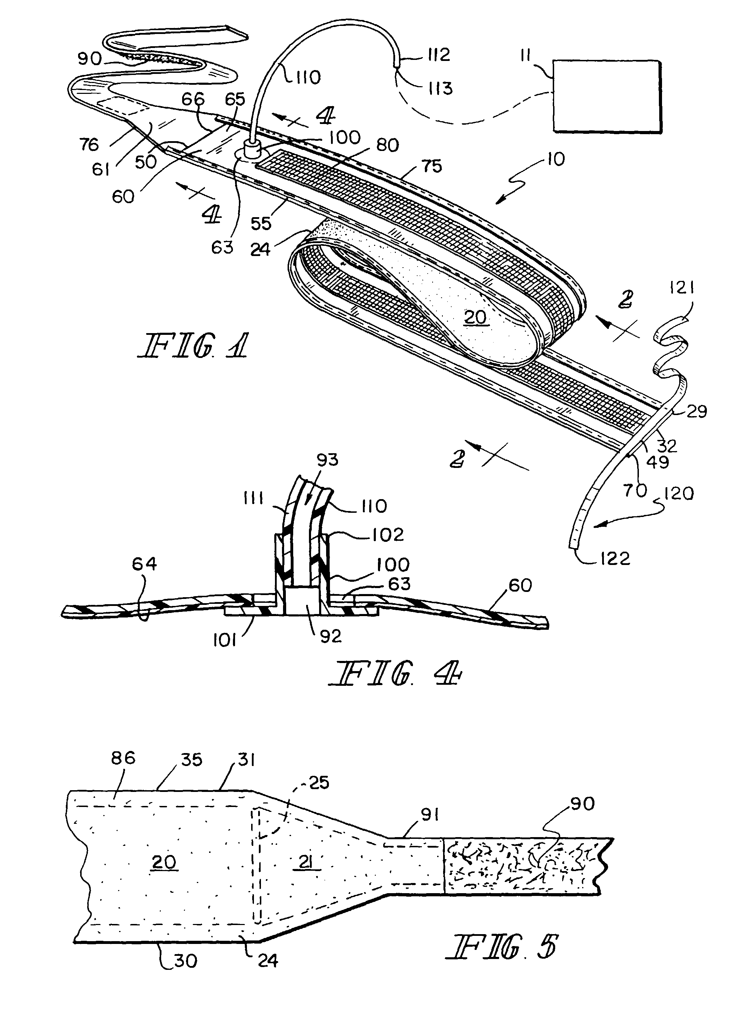

Referring to the drawings, Fig. 1 shows a tourniquet cuff 10, which includes a polyester nap layer 20, an outer nylon layer 60, the hook-portion 80 of a hook-and-loop closure material, a corresponding loop-portion 90, a polyurethane bladder port 100 (best seen in Fig. 4), a PVC tube 110 (best seen in Fig. 4), and a tie 120 having first and second tie ends 121, 122. Polyester nap layer 20 and outer nylon layer 60 are generally rectangular in shape except for a polyester nap funnel-shaped end 21 (Fig. 5) and an outer nylon funnel-shaped end 61, including a funnel perimeter 76.

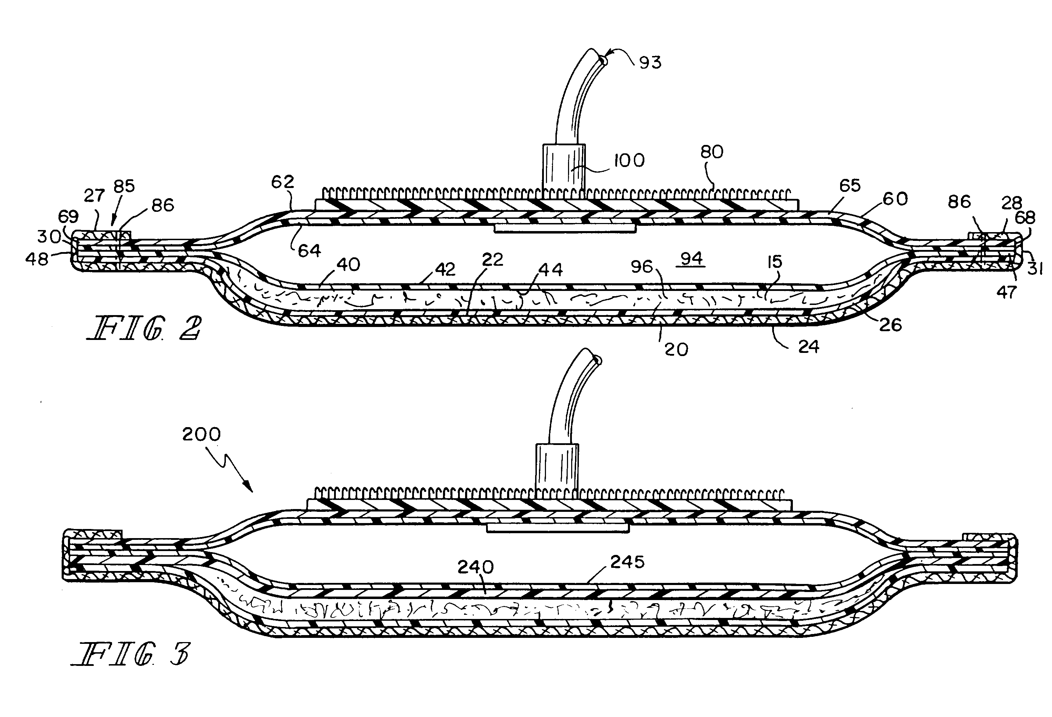

Referring now to Figs. 1, 2, and 5, polyester nap layer 20 includes a first nap side 22, a second nap side 24, and first, second, and third nap fold portions 27, 28, and 29 folded along first, second, and third nap folds 30, 31, and 32. Further, polyester nap layer 20 includes a nap-funnel boundary 25 and a nap perimeter portion 35 located adjacent first, second, and third nap folds 30, 31, 32, a nap funnel boundary 25, and a funnel-shaped end 21. Polyester nap layer 20 may have a felt-like texture which remains exposed on second nap side 24. However, first nap side 22 may be coated with a thin (illustratively 4 mil) layer of polyurethane 26 to produce a surface impervious to the gel-like material 15 (best seen in Fig. 2). It is understood that within the scope of this disclosure, materials other than polyurethane may be used to form such an impervious surface.

As best seen in Fig. 2, adjacent to polyester nap layer 20 is a middle nylon layer 40, including a first middle nylon side 42, a second middle nylon side 44, a first middle nylon edge 47, a second middle nylon edge 48, a third middle nylon edge 49, a fourth middle nylon edge 50, and a middle nylon perimeter portion 55 located adjacent to the first, second, third, and fourth middle nylon edges 47, 48, 49 and 50. First middle nylon side 42 and second middle nylon side 44 is be coated with thin (illustratively 4 mil) layers of polyurethane to produce air-impenetrable surfaces. Again, it is understood that materials other than polyurethane may be used to form such an air-impenetrable surface and that materials other than polyurethane-coated nylon may be used for the middle nylon layer. Middle nylon layer 40 is the same, generally rectangular, shape as polyester nap layer 20 and outer nylon layer 60, except that middle nylon layer 40 does not include a funnel-shaped end. Further, middle nylon layer 40 is slightly smaller than polyester nap layer 20, so that when second middle nylon side 44 is positioned adjacent first nap side 22 and outer nylon layer 60 is positioned adjacent to middle nylon layer 40, first, second and third nap fold portions 27, 28, 29 extend beyond first, second, and third middle nylon edges 47, 48, 49 and polyester nap funnel-shaped end 21 extends beyond fourth middle nylon edge 50. As will be seen below, this arrangement allows formation of the tourniquet cuff 10 in the preferred embodiment.

As shown in Fig. 2, adjacent to middle nylon layer 40 is outer nylon layer 60, which includes a main outer nylon body 65, a first outer nylon side 62, a second outer nylon side 64, a first outer nylon edge 68, a second outer nylon edge 69, and a third outer nylon edge 70. Further, outer nylon layer 60 includes an outer nylon funnel boundary 66 between main outer body 65 and outer nylon funnel-shaped end 61, and an outer perimeter portion 75 located adjacent first, second, and third outer nylon edges 68, 69, and 70 and outer nylon funnel boundary 66 (best seen in Fig. 1.). As with sides 22, 42, and 44, second outer nylon side 64 is coated with a thin (illustratively 4 mil) layer of polyurethane to produce an air-impenetrable surface, but it is understood that materials other than polyurethane may be used to form such an air-impenetrable surface. Outer nylon layer 60 is the same, generally rectangular, shape as polyester nap layer 20, including outer nylon funnel-shaped end 61 which is the same shape and size as polyester nap funnel-shaped end 21. However, outer nylon layer 60 is slightly narrower and shorter than polyester nap layer 20, so that first, second, and third nap fold portions 27, 28, 29 extend beyond first, second, and third outer nylon edges 68, 69, and 70. Lastly, an aperture 63 is formed in outer nylon layer 60 (best seen in Fig. 4).

Referring to Figs. 1 and 4, polyurethane bladder port 100 includes a bladder flange 101 and a bladder nozzle 102. Bladder nozzle 102 is inserted through aperture 63 in outer nylon layer 60 so that bladder flange 101 abuts second outer nylon side 64. Bladder flange 101 is affixed to second outer nylon side 64, coupling bladder port 100 to outer nylon layer 60. As best seen in Figs. 1, 2, and 4, with bladder port 100 coupled to outer nylon layer 60, second outer nylon side 64 is positioned adjacent first middle nylon side 42 so that first, second, and third middle nylon edges 47, 48, 49 line up with first, second, and third outer nylon edges 68, 69, 70, and outer perimeter portion 75 is positioned adjacent middle nylon perimeter portion 55. First nap side 22 is then positioned adjacent second middle nylon side 44 so that first, second, and third nap folds 30, 31, 32 line up with first, second, and third middle nylon edges 47, 48, 49, and first, second, and third outer nylon edges 68, 69, 70. Thus, nap perimeter portion 35 is positioned adjacent middle nylon perimeter portion 55. Therefore, in the illustrated embodiment, middle nylon layer 40 is sandwiched between polyester nap layer 20 and outer nylon layer 60. With the three layers 20, 40, and 60 in position, heat and pressure is applied to nap perimeter portion 35, middle nylon perimeter portion 55, and outer nylon perimeter portion 75 simultaneously, creating a heat seal 85 binding the three layers 20, 40, and 60 together. However, it is understood that other methods of binding the layers together are possible within the scope of this invention. Further, heat seal 85 is air-impenetrable so that an air chamber 94 is created between first middle nylon side 42 and second outer nylon side 64. Bladder port 100 provides an opening 92 (best seen in Fig. 4) which defines a passageway 93 into air chamber 94. However, other than opening 92, air chamber 94 is air-tight. Heat seal 85 also creates a gel chamber 96 between first nap side 22 and second middle nylon side 44. Prior to binding layers 20, 40, and 60 together, a gel-like material 15 is positioned within gel chamber 96. Therefore, when layers 20, 40, and 60 are bound together, gel-like material 15 is sealed within gel chamber 96.

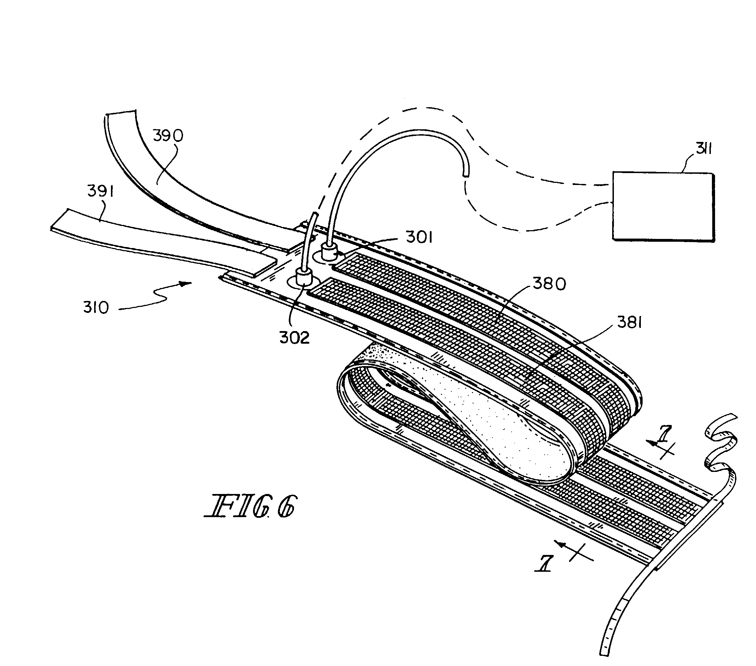

In an alternative embodiment of the tourniquet cuff 200 (Fig. 3), the middle nylon layer 40 is replaced by multiple layers 240 and 245, one cooperating with polyester nap layer 20 to create gel chamber 96 and another cooperating with outer nylon layer 60 to create air chamber 94. Also, in another alternative embodiment, gel-like material 15 is presealed within its own flexible packaging (not shown), and the entire gel-like material package is shaped and positioned within gel chamber 96. In this embodiment, gel chamber 96 need not be sealed, thus allowing easy removal and replacement of the gel-like material package.

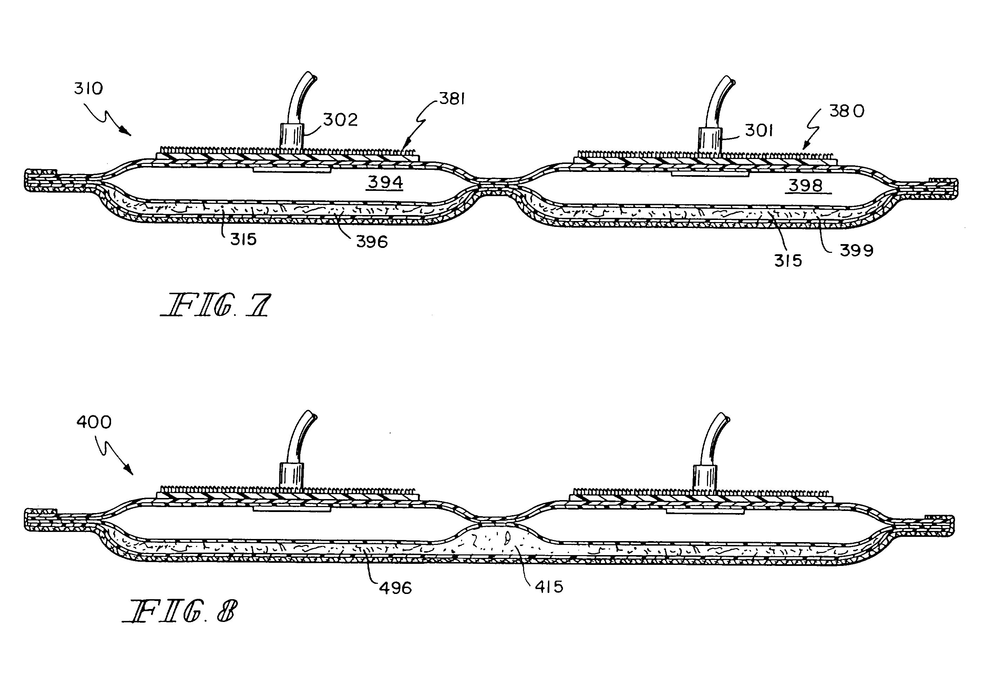

In a further embodiment, as shown in Figs. 6 and 7, a gel-like material 315 is used in a tourniquet cuff of a Bier's Block design 310, in which the tourniquet has a first air bladder 394 having a first bladder port 302 and a second air bladder having a second bladder port 301 placed side-by-side. Adjacent to first air bladder 394 is placed a first gel cavity 396 with a gel-like material 315 disposed therein. Likewise, adjacent to second air bladder 398 is placed a second gel cavity 399 with gel-like material 315 disposed within. It is understood within the scope of this disclosure that the gel-like material in first gel cavity 394 need not be the same as gel-like material in the second gel cavity 396. It is also understood within the scope of this disclosure that funnel-shaped end 16 as shown in Fig. 1, for example, could be adapted to the instant embodiment.

In still another embodiment 400 (Fig. 8), first and second gel cavities 396 and 399 (shown in Fig. 7) are replaced by a single gel cavity 496. As with the other embodiments described herein, the gel-like material is placed between air bladders 394 and 398 and the patient's limb.

Referring back to Figs. 1, 2, 4, and 5, once polyester nap layer 20, middle nylon layer 40, and outer nylon layer 60 have been heat-sealed together, first nap fold portion 27, second nap fold portion 28, and third nap fold portion 29 are folded over first outer nylon side 62 along first, second, and third nap folds 30, 31, and 32, respectively. Once nap fold portions 27, 28, 29 are folded over first outer nylon side 62, nap fold portions 27, 28, 29 is sewn through heat seal 85 with a stitching 86. Other methods of affixing nap fold portions 27, 28, 29 may be used.

As best seen in Figs. 1 and 5, a first end 91 of the loop-portion 90 of a hook-and-loop closure material is positioned between outer nylon funnel-shaped end 61 of outer nylon layer 60 and polyester nap funnel-shaped end 21 of polyester nap layer 20. Illustratively, stitching 86 continues from nap fold portions 27, 28, 29, as described above, along the funnel perimeter 76 of outer nylon funnel-shaped end 61 and polyester nap funnel-shaped end 21, thereby coupling outer nylon funnel-shaped end 61, polyester nap funnel-shaped end 21 and end 91 of the loop-portion 90. Fixed to main outer nylon body 65 on first outer nylon side 62 is a length of the corresponding hook-portion 80 of the hook-and-loop closure material. A tie 120 is fixed to first and second nap fold portions 27 and 28 adjacent and parallel to third nap fold portion 29. Lastly, PVC tube 110 includes a first PVC end 111 fixed to bladder nozzle 102 and a second PVC end 112 fixed to a connector 113. An inflation device 11 provides a means for inflating air chamber 94.

During use, tourniquet cuff 10 is wrapped around a patient's limb, with polyester nap layer 20 adjacent the patient's limb, so that the loop-portion 90 of the hook-and-loop closure material engages the hook-portion 80 securing tourniquet cuff 10 to the patient's limb. Tie ends 121, 122 of tie 120 are then tied together so that tie 120 is secured around the loop-portion 90. Air chamber 94 is then be inflated by pumping air through PVC tube 110, through passageway 93 in bladder port 100, and into air chamber 94. With tourniquet cuff 10 wrapped around the patient's limb, gel chamber 96 is positioned between air chamber 94 and the patient's limb. Therefore, when air is pumped into air chamber 94, pressure is exerted around gel chamber 96 which, in turn, transmits pressure around the patient's limb. While a hook-and-loop closure material such as that sold under the trade mark VELCRO is utilized in the preferred embodiment, other methods, including straps, buckles, snaps, and tape, may be used to secure the tourniquet around a patent's limb.

The gel-like material may be any suitable material that will uniformly distribute the pressure on the patient's limb. Suitable materials are sold under the trade marks FLOAM (by TekSource Inc of Draper, Utah), FLO-LITE (by Alden Laboratories Inc of Boulder, Colorado), and ISOGEL (by Pittsburgh Plastics Inc of Zellenople, Pennsylvania).

## Claims

1. A tourniquet cuff comprising:
an elongated, inflatable cavity formed to be secured about a patient's limb;
an elongated gel-filled cavity coupled to the inflatable cavity, the gel-filled cavity being disposed between the inflatable cavity and the patient's limb; and
means for adjustably securing the tourniquet about the patient's limb.

2. A cuff as claimed in claim 1, in which the elongated, inflatable cavity has a first end, a second end, a top side, and a bottom side, the elongated gel-filled cavity is attached to the bottom side, and in which the means for adjustably securing the tourniquet comprises:
a tapered portion having a wide end and a narrow end, the wide end being coupled to the first end of the elongated, inflatable cavity;
the loop-portion of a hook-and-loop closure material coupled to the narrow end of the tapered portion; and
the corresponding hook-portion of the hook-and-loop closure material coupled to the top side of the elongated inflatable cavity.

3. A tourniquet cuff comprising:
a first layer having an inner and an outer side, the first layer being impermeable to air;
a second layer having a first and a second side and being impermeable to air, the first layer and the second layer being coupled together to define a first cavity between the inner side of the first layer and the first side of the second layer;
a gel-like material disposed within the first cavity to uniformly and comfortably distribute pressure on a patient's limb;
a third layer having an inner and an outer side and being impermeable to air, the second layer and the third layer being coupled together to define a second cavity between the second side of the second layer and the inner side of the third layer, the second cavity being inflatable; and
means for adjustably securing the tourniquet about the patient's limb with the outer side of the nap material in contact with the patient's limb.

4. A cuff as claimed in claim 3, in which the third layer has a tapered end.

5. A cuff as claimed in claim 3, in which the inner side of the first layer is coated with polyurethane.

6. A cuff as claimed in claim 3, in which the second layer is formed of polyurethane.

7. A cuff as claimed in claim 3, in which the inner side of the third layer is coated with polyurethane.

8. A cuff as claimed in claim 3, in which the second layer has an aperture through which a pressurizing fluid may be introduced.

9. A cuff as claimed in claim 3, in which the means for securing the tourniquet comprises a hook-and-loop closure material.

10. A tourniquet cuff for reducing blood flow in a patient's limb, which comprises:
a length of polyester nap material having an inner and an outer side;
a first nylon layer having a first and a second side, the polyester nap material and the first nylon layer attached together to define a first cavity between the inner side of the polyester nap material and the first side of the first nylon layer;
a gel-like material disposed within the first cavity;
a second nylon layer having a first and a second side, the first nylon layer and the second nylon layer attached together with the second side of the first nylon layer facing the first side of the second nylon layer;
a third nylon layer having an inner and an outer side, the second nylon layer and the third nylon layer being coupled together to define a second cavity between the second side of the second nylon layer and the inner side of the third nylon layer, the second cavity being inflatable; and
means for adjustably securing the tourniquet about the patient's limb with the outer side of the polyester nap material in contact with the patient's limb.

11. A cuff as claimed in claim 10, in which the third nylon layer has a tapered end.

12. A cuff as claimed in claim 10, in which the third nylon layer has an aperture through which a pressurizing fluid may be introduced.

13. A tourniquet cuff comprising:
an elongated inflatable chamber to be wrapped and fastened about a patient's limb and inflated to apply pressure to the limb and reduce or eliminate blood flow;
an additional chamber attached to the inflatable chamber to extend along it and to be disposed between the inflatable chamber and the surface of the patient's limb; and
a gel-like substance disposed within the additional chamber to uniformly and comfortably distribute pressure on the patient's limb.

14. A cuff as claimed in claim 13, in which a funnel-shaped portion of nylon material is coupled to the elongated inflatable chamber.
